# EUROPEAN PATENT APPLICATION

(11) **EP 4 623 864 A1**
(43) Date of publication of application: **01.10.2025**
(21) Application number: 25166465.2
(22) Date of filing: 26.03.2025
(51) Int. Cl.: A61F 2/07, A61F 2/24, A61F 2/06

(54) **PROSTHETIC VALVE FOR TREATMENT OF TRICUSPID VALVE INSUFFICIENCY**

(30) Priority: 27.03.2024 DE 102024108793
(71) Applicant: MEDIRA GmbH, 72336 Balingen (DE)
(72) Inventor: PISANI, Giuseppe, 72336 Balingen (DE); MOGENTALE, Davide, 72336 Balingen (DE); BOGENSCHÜTZ, Thomas, 72379 Hechingen (DE)
(74) Representative: Witte, Weller & Partner Patentanwälte mbB

(57) **Abstract**

The present invention relates to a device for implantation into the heart of a mammal under longitudinal and rotational freedom of orientation and is intended for treating tricuspid regurgitation. The prosthetic valve device comprises a tubular stent to be implanted into the *superior vena cava* and *inferior vena cava* and at least one valve allowing unidirectional blood flow into the right atrium.

## Description

The present invention relates to a prosthetic valve device for treatment of a valve insufficiency of a heart, in particular of insufficiency of a tricuspid valve, having a longitudinal axis or length l, a proximal portion, a medium portion and a distal portion, and a lumen permitting blood flow there through.

The human heart is subdivided by septa into right and left halves, and a constriction subdivides each half of the organ into two cavities, the upper cavity being called the atrium, the lower the ventricle, respectively. Thus, the heart consists of four chambers, i.e., right and left atria, and right and left ventricles. Via the four valves of the human heart, i.e. the aortic, mitral, tricuspid and pulmonary valves, a one-way blood flow through the (healthy) heart is maintained. Thus, the four heart valves make sure that blood always flows freely in a forward direction and that there is no backward leakage.

In the heart, blood flows from the right and left atria into the ventricles through the open tricuspid and mitral valves. When the ventricles are full, the tricuspid and mitral valves shut. This action, i.e. the closing of the tricuspid and the mitral valve, prevents blood from flowing backward into the atria while the ventricles contract. As the ventricles begin to contract, the pulmonic and aortic valves are forced open, thus pumping blood out of the ventricles: Blood present in the right ventricle passes through the open pulmonic valve into the pulmonary artery, and blood present in the left ventricle passes through the open aortic valve into the aorta where it is delivered to the rest of the body. When the ventricles finish contracting and begin to relax, the aortic and pulmonic valves shut. These valves prevent blood from flowing back into the ventricles.

With each heartbeat, this pattern is repeated, causing blood to flow continuously to the heart, lungs, and body. Due to their vital function, diseased or malfunctioning heart valves are a major threat for a person's life.

Several different types of valve disorders are known, such as, e.g., stenosis, which occurs when a heart valve doesn't fully open due to stiff or fused leaflets preventing them from opening properly, or prolapse, where the valve flaps do not close smoothly or evenly but collapse backwards into the heart chamber they are supposed to be sealing off.

Valve regurgitation (backward flow) is also common problem, and occurs when a heart valve doesn't close tightly, as a consequence of which the valve does not seal and blood leaks backwards across the valve. This condition - also called valvular insufficiency - reduces the heart's pumping efficiency: When the heart contracts blood is pumped forward in the proper direction but is also forced backwards through the damaged valve. As the leak worsens, the heart has to work harder to make up for the leaky valve and less blood may flow to the rest of the body. Depending on which valve is affected, the condition is called tricuspid regurgitation, pulmonary regurgitation, mitral regurgitation, or aortic regurgitation.

While mitral insufficiency has - likely due to its higher occurrence - been subject matter of many treatment approaches in recent years, tricuspid insufficiency, or rather its treatment has gained only little attention over the past years. Tricuspid insufficiency may be asymptomatic, however, common symptoms are, e.g. hepatomegaly, edema and jugular distenosis. As a result of the failure of the tricuspid valve to close properly, with each heart beat some blood passes from the right ventricle to the right atrium, the opposite of the normal direction. Although congenital causes of tricuspid insufficiency exist, most cases are due to annulus dilation and dilation of the right ventricle, and this dilation leads to a derangement of the normal anatomy and mechanics of the tricuspid valve and the muscles governing its proper function. The result is incompetence of the tricuspid valve.

However, isolated surgical tricuspid valve repair is seldomly performed, and remains rather undertreated. Actually, most repairs are performed in the context with other planned cardiac surgeries.

The main therapy of tricuspid insufficiency is treatment of underlying cause, which is why in most cases surgery is not indicated since the root problem lies with a dilated or damaged right ventricle. Medical therapy with diuretics is the mainstay of treatment. Unfortunately, this can lead to volume depletion and decreased cardiac output. Indeed, one must often accept a certain degree of symptomatic tricuspid insufficiency in order to prevent a decrease in cardiac output. Treatment with medicines to reduce cardiac afterload may also be of benefit but a similar risk of depressed cardiac output applies.

Human heart valves may be replaced with mechanical valves, or with specially prepared heart valves from human or animal donors (known as bioprosthetic or tissue valves). Bioprosthetic valves are sometimes called tissue valves and made from specially treated natural ("biological") valves. These valves come from two sources: human donors and animals. Valves from animal sources (usually cows or pigs) are very similar to those found in the human heart.

Surgical repair or replacement of the tricuspid valve carries a high operative mortality. When applying surgical means, tricuspid regurgitation is rectified either by replacement of the total valve with a replacement valve or by constriction of the valve ring with an annular remodeling ring, which involves rigid or flexible annular bands, which are intended to reduce annular size.

Due to the high risk of surgical operations and due to the fact, that in many cases a surgery is even impossible to perform, e.g. if the patient is inoperable or operable only at a too high surgical risk, transcatheter techniques and devices for tricuspid regurgitation treatment have recently been developed; however, only limited experimental transcatheter data is available.

E.g., WO 2012/018599 A1 discloses a two valve caval stent for functional replacement of an incompetent tricuspid valve, which may be delivered by transcatheter placement. The uncovered device comprises two stents connected by a bridge spanning the right atrium, and two valves anchored by the stents in the *superior* and *inferior vena cava.*

Further, WO 2004/093638 discloses a device and methods for treatment of tricuspid regurgitation, where a first and a second stented valve are implanted at the *superior vena cava* and *inferior vena cava.* The device is intended to permit blood flow towards the right atrium of a patient and prevent blood flow in the opposite direction.

Nevertheless, the currently available two-partite devices and their handling imply complicated basically two-partite deployment methods, making a smooth and fast valve replacement difficult to achieve.

Considering this, EP 2 929 860 A1 discloses a single partially covered tubular caval stent to be placed simultaneously into the *superior vena cava* and *inferior vena cava* comprising a single valve placed within a hole of the tubular stent. However, providing a single non-circumferential, but punctual valve structure within the stent generates another type of drawback, which is the necessity to actively orientate the device such that the valve is positioned towards the atrium to allow proper blood efflux from the lumen of the stent.

Thus, it is an object of the present invention to provide for a device that facilitates the treatment of tricuspid regurgitation and overcomes the drawbacks of the prior art devices and treatment methods.

According to the invention, this and other objects is solved by a prosthetic heart valve device (i.e., "prosthetic valve device" as used herein) for treatment of tricuspid valve insufficiency of a heart of a mammal, the prosthetic valve device comprising a length l, a proximal portion for anchoring the prosthetic valve device in the superior vena cava, a distal portion for anchoring the prosthetic valve device in the inferior vena cava, and a medium portion interposed between the proximal and the distal portion, a stent frame extending from the proximal portion over the medium portion to the distal portion, wherein the proximal portion and the distal portion are tubular and have a respective lumen, and wherein in the proximal portion and the distal portion the stent frame is covered by prosthetic material at least partially, and wherein the prosthetic valve device further comprises a first valve being attached within the lumen of the proximal portion, and optionally a second valve being attached within the lumen of the distal portion; the medium portion is circumferentially free from prosthetic material, thus forming an uncovered section, and wherein the medium portion, in an implanted state of the prosthetic valve device, is configured for being positioned within the right atrium of the heart, and for allowing blood flow out from the lumen into the right atrium.

With the prosthetic valve device according to the invention and its use in the treatment of tricuspid insufficiency, it is possible to securely and conveniently support/integrate the tricuspid valve's function and effectively support the heart's function. The prosthetic valve device according to the invention spans a path through the heart from the *superior vena cava* to the *inferior vena cava* while simultaneously guaranteeing, by means of a fully functioning first and any further valve attached in the lumen of the covered portions of the stent frame, that the one-way blood flow from the venous system through the right atrium to the right ventricle is maintained, and a backflow of the blood from the right ventricle into the venous system can be prevented, thus effectively treating the tricuspid insufficiency-related complications.

According to the invention, the prosthetic valve device of the invention is configured and designed such, that the first valve, which is attached within the lumen of the proximal portion, can, thus, get positioned within the *superior vena cava,* and the optional second valve, which is optionally attached within the lumen of the distal portion, can, thus, get positioned within the *inferior vena cava* upon implantation of the prosthetic valve device into the *vena cava* of a patient to be treated.

The prosthetic valve device according to the invention is easy to handle and to deploy, since only one device needs to be deployed - contrary to other devices currently available which either necessitate the deployment of two separate valves in the *superior vena cava* and *inferior vena cava,* or which apply a replacement annular ring of the natural valve. The specific configurations of the prosthetic valve device according to the invention allow an implantation with a certain degree of longitudinal as well as full rotational freedom.

Thus, with the prosthetic valve device according to the invention, the overall deployment and valve replacement procedure, and thus, the overall surgical operation, can be fast and easily accomplished.

Presently, and as generally understood, a "stent frame" is to be understood and referred to as a generally cylindrical or tubular, radially-expandable support frame or body and means any device or structure that adds rigidity, expansion force, or support to a prosthesis, while "stent graft" or "stent prosthesis" refers to a prosthesis comprising a stent and a prosthetic (graft) material associated therewith that forms a fluid-tight or substantially fluid-tight lumen through at least a portion of its length. The cylindrical/tubular body of stents/stent grafts is inserted into the vessel/organ to be treated and is expanded or self-expandable and fixed or fixes itself at the appropriate site in order to keep open the lumen of the vessel/organ.

Accordingly, a "graft" or prosthetic material is a cylindrical liner that may be disposed on the stent's interior, exterior or both. A wide variety of attachment mechanisms are available to join the stent and prosthetic/graft material together, including but not limited to, sutures, adhesive bonding, heat welding, and ultrasonic welding. Presently, a "covering" also may designate or is designating a prosthetic/graft material attached to a stent member, which is why a "stent graft" is presently, and throughout the relevant field, also designated as "covered stent" or "covered stent graft".

A stent frame generally is generally made from one or several stent wires made of a self-expanding or expandable material, optionally formed in stent rings, or the stent frame is laser-cut. The stent rings, or, respectively, the wire framework can be connected to each other directly via struts, or indirectly via a prosthetic material/graft.

The stent rings of the stent graft may represent single metal rings forming a metal mesh, the rings circumferentially meandering and being disposed successively in the graft member's longitudinal axis/direction, wherein the metal rings have a Z-shaped profile with pointed arches pointing alternately toward the proximal end and distal end of the prosthetic valve device.

According to a preferred embodiment, stent rings are arranged over the length l, such, that the pointed arches of a first stent ring, which point towards the proximal direction, are aligned with those pointed arches of a second stent ring, which also point towards the proximal direction, wherein the first and second stent ring are adjacent to one another and arranged at a certain distance DS from each other. Accordingly, in a preferred embodiment, at least a first and a second stent ring do not directly touch each other and are not interconnected with one another. According to a preferred embodiment, all stent rings are arranged in a certain distance DS from each other.

Thus, and as generally stent devices, the prosthetic valve device of the invention is expandable from a compressed state to an expanded state, wherein the prosthetic valve device is in the expanded state when implanted for treatment into the heart of a mammal.

The prosthetic valve device of the invention can be a single substantially uniform continuous and tubular main body deployable over its full length I within a single deployment process. During this deployment, both the proximal and the distal portion of the main body are placed at their respective position and released from a deployment catheter one after the other.

According to the invention, in the proximal portion and the distal portion the stent frame is covered by prosthetic material, at least partially, preferably circumferentially totally covered in the proximal and distal portion, wherein in the medium portion the stent frame is uncovered, i.e. circumferentially free from prosthetic material. According to the invention the prosthetic valve device is configured such, that the uncovered medium portion of the stent frame, upon placement and expansion in the heart of a mammal, is located within the atrium and fully circumferentially uncovered (or free from) by prosthetic material to ensure an undirected blood efflux from the lumen of the stent frame/ the prosthetic valve device due to its direct fluid-communication with the atrium.

Proximal (i.e. towards the proximal direction and towards the *superior vena cava*) to the medium portion, the proximal portion is provided. The proximal portion is circumferentially covered by prosthetic material such that the cover provides for a fluid-tight or substantially fluid-tight sealing between the prosthetic valve device and the surrounding tissue of the *superior vena cava.* In other words, the prosthetic valve device is configured, such, that the covering provided by the prosthetic material does span the atrium.

According to the invention, a first valve being attached within the lumen in the proximal portion is provided. The section of the proximal portion located proximal to the first valve is fluid-tight or substantially fluid-tight sealed by the first valve when the blood pressure inside the atrium is higher than in the *superior vena cava.* This way, between the atrium and the *superior vena cava,* only unidirectional blood flow towards the right ventricle occurs, thus, limiting the venous backflow caused by the tricuspid insufficiency.

It is to be noted that in other embodiments more valves can be provided within the proximal portion, while the first valve is always the most distal one.

Distal to the medium portion, i.e. into the distal direction towards the *inferior vena cava,* the distal portion is provided. The distal portion is circumferentially covered by prosthetic material such that the cover provides for a fluid-tight or substantially fluid-tight sealing between the prosthetic valve device and the surrounding tissue. In other words, the prosthetic valve device is configured, such, that the covering provided by the prosthetic material does span the atrium.

According to the invention, optionally a second valve is provided being attached within the lumen in the distal portion. By means of the second valve, a section of the distal portion located distal to the second valve is fluid-tight or substantially fluid-tight sealed when the blood pressure inside the atrium is higher than in the *inferior vena cava.* This way, between the atrium and the *inferior vena cava,* only unidirectional blood flow towards the right ventricle occurs, thus, limiting the venous backflow caused by the tricuspid insufficiency.

It is to be noted that in other embodiments more valves can be provided within the distal portion, while the second valve is always the most proximal one.

The stent frame can be made of or comprise any suitable material, including but not limited to biocompatible metals, implantable quality stainless steel wires, nickel, and titanium alloys, in particular nitinol, and biocompatible plastics attached to a graft.

In an aspect of the invention, the stent frame comprises or consists of a laser-cut stent frame, individual stent rings, or a woven or braided stent frame comprising interwoven or braided stent elements. In an aspect of the invention, the stent frame is tubular and flexible. In a further aspect, the stent frame and is either self-expandable or balloon-expandable, which allows for radial force fixation within the *vena cava.*

According to one embodiment of the invention the stent frame has plurality of individual stent rings, preferably 4, 5, 6, 7, 8, 9 or 10 individual stent rings, which are successively arranged over the length l, or only in the proximal and distal portion, and meander circumferentially. In an embodiment of the prosthetic valve device, the individual stent rings can be directly interconnected with one another via struts, and/or indirectly via prosthetic material.

The prosthetic device, in the distal portion of the stent frame, can or cannot comprise a valve, i.e. a second valve, which can thus be optional. Also, in an aspect of this particular embodiment, the distal portion can be covered or uncovered. In the embodiment, where the distal portion does not comprise a valve (i.e., where the prosthetic valve device of the invention does only comprise a first valve in the proximal portion), a second valve/valve prosthesis may be separately/in a second step introduced into the distal portion, i.e. after/after in a first step the prosthetic valve device has been placed into the heart of the patient/mammal.

According to another embodiment of the invention the stent frame has from 4, 5, 6, 7, 8, 9 or 10 individual stent rings, which are successively arranged over the length l of the main body, or only in the distal and proximal portion, and which meander circumferentially, and wherein the individual stent rings in the proximal and distal portion are not directly interconnected with one another by struts, and wherein only the stent rings in the medium portion are interconnected with one another via struts. Within the proximal and distal portion, the prosthetic material provides for an indirect connection of the individual rings. In this way, the main body gains flexibility and thus facilitated compliance with the movements of the surrounding tissue is achieved.

In yet another embodiment, the individual rings in the proximal portion are not interconnected by stent structures, while only the individual rings in the distal portion and the medium portion are interconnected.

In a preferred embodiment, the uncovered section of the medium portion is formed by at least two stent rings that are positioned at a distance from each other in the medium portion and that are interconnected via struts, such, that open cells between the interconnected stent rings are formed in the medium portion, wherein the open cells are dimensioned and sized to allow blood flow out from the lumen into the right atrium. Also, in this embodiment, the proximal and the distal portion comprise a plurality of individual stent rings, preferably 4, 5, 6, 7, 8, 9 or 10 individual stent rings, which are successively arranged over the respective length of the proximal and distal portion, and which stent rings are not interconnected with one another via struts, and which proximal and distal portion are covered by prosthesis material.

It has shown to be preferable that the at least two individual rings in the medium portion that are interconnected via at least two, preferably 2, 3, 4, 5, 6, 7 or 8 struts are circumferentially meandering such, that they have a Z-shaped profile with pointed arches pointing alternately towards the proximal portion and the distal portion of the prosthetic valve device, and that the struts in the medium section connect an arch of a first stent ring pointing towards the distal portion with an arch pointing towards the proximal portion of a second stent ring, which second stent ring is arranged distally from the first stent ring.

In other words, via the interconnection of the two stent rings in the medium portion via the struts, at least two non-planar hexagonal openings or stent cell structures are formed.

Thus, according to an embodiment, the medium portion of the stent device has at least two, preferably 2, 3, 4, 5, 6, 7 or 8 irregular or regular non-planar hexagonal openings/stent cell structures. "Regular", in this connection, is to be understood, such, that each of the six sides (i.e. stent struts) of the hexagonal stent structure are equal in length, while "irregular" is to be understood, such, that not all of the stent struts/sides are equal in length. The plane of the hexagonal stent structures is not exactly flat, but bended, depending on the number of hexagonal stent structures employed, such that the entity of hexagonal stent structures forms a tubular stent structure. In other words, the proximal and the distal end of the cell structures /openings mimic the Z-shaped profile of the individual rings with pointed arches pointing alternately towards the proximal portion and the distal portion of the prosthetic valve device, and the connected legs of the hexagonal stent structures mimic the struts in the medium portion connecting the individual rings.

In this embodiment, also other stent rings of the stent frame can be interconnected by struts, i.e. stent rings in the proximal and/or distal portion, or they can be interconnected exclusively by the prosthetic material attached to the proximal or distal stent frame. In any case the openings formed by the hexagonal stent cell structures are uncovered. Thus, the medium portion is in fluid-communication with the atrium.

Accordingly, in a particularly preferred embodiment, the prosthetic valve device according to the invention is a prosthetic valve device for treatment of tricuspid valve insufficiency of a heart of a mammal, the prosthetic valve device comprising a length I, a proximal portion for anchoring the prosthetic valve device in the *superior vena cava,* a distal portion for anchoring the prosthetic valve device in the *inferior vena cava,* and a medium portion interposed between the proximal and the distal portion, a stent frame extending from the proximal portion over the medium portion to the distal portion, wherein the proximal portion and the distal portion are tubular and have a respective lumen, and wherein in the proximal portion and the distal portion the stent frame is covered by prosthetic material at least partially or completely, wherein the proximal and distal portion consists of individual, i.e. separate, circumferentially meandering stent rings, the stent rings not being interconnected with one another via struts, which circumferentially meandering stent rings have a Z-shaped profile with pointed arches pointing alternately toward the proximal end and distal end of the prosthetic valve device, wherein the medium portion is circumferentially free from prosthetic material, i.e. not circumferentially covered by prosthetic material, thus forming an uncovered section, and wherein the medium portion, in an implanted state of the prosthetic valve device, is configured for being positioned within the right atrium of the heart, and for allowing blood flow out from the lumen into the right atrium, which uncovered section of the medium portion is formed by two stent rings that are positioned at a distance from each other in the medium portion and that are interconnected via struts, such, that open cells between the interconnected stent rings are formed in the medium portion, wherein the open cells are dimensioned and sized to allow blood flow out from the lumen into the right atrium, and preferably have a non-planar hexagonal shape, wherein the two individual rings in the medium portion that are interconnected via at least two, preferably 2, 3, 4, 5, or 6 struts are circumferentially meandering such, that they have a Z-shaped profile with pointed arches pointing alternately towards the proximal portion and the distal portion of the prosthetic valve device, and that the struts in the medium section connect an arch of a first stent ring pointing towards the distal portion with an arch pointing towards the proximal portion of a second stent ring, which second stent ring is arranged distally from the first stent ring, and wherein the prosthetic valve device further comprises a first valve being attached within the lumen of the proximal portion, and optionally a second valve being attached within the lumen of the distal portion.

According to another embodiment of the prosthetic valve device of the invention, the stent frame is formed of a plurality of single (or individual) straight, non-circumferentially-meandering stent wires extending from the proximal portion to the distal portion, wherein the stent wires, in the medium portion are being held together via a sleeve, and wherein the single (or individual) stent wires, in the proximal portion and the distal portion form bent loop-portions, respectively, which are arranged to form the tubular shape of the proximal and distal portion.

In this embodiment, the medium portion of the stent frame is represented by straight or unbent (as contrary to circumferentially meandering or Z-shaped) stent wires, which are bundled together, and thus, contact each other, via a sleeve surrounding and holding together the straight stent wires. The stent wires, in the medium portion, can extend substantially parallel to the longitudinal length l of the prosthetic valve device. The proximal and the distal portions, via the bent loop-portions, represent the tubular covered portions respectively carrying a valve within their respective lumen, or where only the proximal portion is carrying a valve. In that way, the uncovered medium portion also allows blood flow out from the lumen of the proximal and distal portion into the right atrium. Further, in this embodiment, and in other words, the single, individual straight stent wires, via the means of the sleeve and in the portion of the sleeve, contact each other, such, that they are directly adjacent to one another in the portion where they are bundled/held through the sleeve.

According to an embodiment, between 3 to 8 preferably between 3 to 6, stent wires are provided, which extend into the proximal and/or distal direction, and, in the proximal and distal portion form the loop-portions. In a preferred aspect of this embodiment, the stent wires respectively extend only in the proximal and only the distal direction, or, in other words, there is a first plurality of stent wires extending in the proximal direction P and a second plurality of stent wires extending in the distal direction D. In this embodiment, the respective ends of the respective first and second plurality of stent wires are attached within the sleeve, such, that their length in both directions, i.e. the proximal and distal direction, can be adapted through the sleeve. Thus, preferably, between 3 and 8, or 3, 4, 5, 6, 7, 8 stent wires extend into the proximal direction, and between 3, and 8, or 3, 4, 5, 6, 7, 8 stent wires extend into the distal direction, with the respective ends of the stent wires being attached to the sleeve.

In a preferred aspect of this embodiment, the stent wires extend, in the medium portion, in a first section A substantially parallel to the longitudinal axis or length l of the prosthetic valve device 100, and in a second section B at an angle α, outwardly from the longitudinal axis. In another preferred aspect of this embodiment, the stent wires directly extend outwardly from the sleeve at an angle at an angle α, with reference to the longitudinal axis or length l of the prosthetic valve device.

In an aspect of this embodiment, the stent frame further comprises a plurality of individual stent rings circumferentially meandering and being attached to the bent loop-portions in the proximal portion and the distal portion. This additionally supports the structural strength of the proximal and distal portion.

According to another embodiment of the prosthetic valve device, the stent frame in the proximal portion and the distal portion is formed by circumferentially meandering stent rings, optionally interconnected with one another, and the stent frame in the medium portion is formed by straight, non-meandering stent wires being connected with the stent frame of the proximal portion and the distal portion, the stent wires, in the medium portion being held together and attached within via a sleeve.

In this embodiment, the proximal and distal portions are formed from separate stent rings, which are attached to the stent wires extending and forming the medium portion. According to an aspect of this embodiment, the stent wires extending towards the proximal direction are connected to circumferentially meandering stent rings in the proximal and distal portion, which circumferentially meandering stent rings have a Z-shaped profile with pointed arches pointing alternately toward the proximal end and distal end of the prosthetic valve device. Preferably, the stent wires extending towards the proximal portion are attached to the stent ring adjacent to the medium portion, such that the stent wires are attached to pointed arches of the stent ring of the proximal portion, which point into the distal direction. Also preferably, the stent wires extending towards the distal portion are attached to the stent ring adjacent to the medium portion, such that the stent wires are attached to pointed arches of the stent ring of the distal portion, which point into the proximal direction.

According to an aspect of this embodiment, 2, 3, 4, 5, 6, 7, or 8 stent wires are attached, respectively, to 2, 3, 4, 5, 6, 7, or 8 pointed arches pointing in the proximal or distal direction. It is to be understood that, when referring to the stent ring of the proximal portion which is adjacent to the medium portion, the number of pointed arches pointing into the distal direction and the number of stent wires attached thereto does not need to be the same. In other words, e.g., 3 stent wires may be attached to three pointed arches pointing in the distal direction, or 4 stent wires may be attached to four pointed arches pointing in the distal direction, while in each case 2, 3, 4, or 5 pointed arches pointing in the distal direction are not connected with or attached to a stent wire, and which, as a consequence, are "free" pointed arches. The same applies vice versa for the distal portions, i.e. when referring to the stent ring of the distal portion which is adjacent to the medium portion.

Accordingly, the present invention, in a preferred embodiment, concerns a prosthetic valve device for treatment of tricuspid valve insufficiency of a heart of a mammal, the prosthetic valve device comprising a length I, a proximal portion for anchoring the prosthetic valve device in the *superior vena cava,* a distal portion for anchoring the prosthetic valve device in the *inferior vena cava,* and a medium portion interposed between the proximal and the distal portion, a stent frame extending from the proximal portion over the medium portion to the distal portion, wherein the proximal portion and the distal portion are tubular and have a respective lumen, and wherein in the proximal portion and the distal portion the stent frame is covered by prosthetic material at least partially or completely, wherein the medium portion is circumferentially free from prosthetic material, i.e. not circumferentially covered by prosthetic material, thus forming an uncovered section, and wherein the medium portion, in an implanted state of the prosthetic valve device, is configured for being positioned within the right atrium of the heart, and for allowing blood flow out from the lumen into the right atrium, wherein the stent frame is formed of a plurality of single (or individual) straight, non-meandering stent wires extending in the proximal portion and in the distal portion, and wherein the single (or individual) stent wires, in the proximal portion and the distal portion form bent loop-portions, respectively, which bent loop-portions are arranged to form the tubular shape of the proximal and distal portion, and wherein the medium portion of the stent frame is formed by the stent wires being straight or unbent in the medium portion, which stent wires are bundled together in the medium portion via a sleeve surrounding and holding together the stent wires in the medium portion, wherein the stent wires, in the medium portion, extend substantially parallel to the longitudinal length l of the prosthetic valve device, and wherein the proximal and distal portion comprise individual, i.e. separate, circumferentially meandering stent rings, which circumferentially meandering stent rings have a Z-shaped profile with pointed arches pointing alternately toward the proximal end and distal end of the prosthetic valve device, and wherein preferably the stent wires extend, in the medium portion, in a first section A substantially parallel to the longitudinal axis or length l of the prosthetic valve device 100, and in a second section B at an angle α, outwardly from the longitudinal axis.

In a preferred aspect of this embodiment, the stent wires respectively extend only in the proximal and only the distal direction, or, in other words, there is a first plurality of stent wires extending in the proximal direction P and a second plurality of stent wires extending in the distal direction D. In this embodiment, the respective ends of the respective first and second plurality of stent wires are attached within the sleeve, such, that their length in both directions, i.e. the proximal and distal direction, can be adapted through the sleeve. Thus, preferably, between 3 and 8, or 3, 4, 5, 6, 7, 8 stent wires extend into the proximal direction, and between 3, and 8, or 3, 4, 5, 6, 7, 8 stent wires extend into the distal direction, with the respective ends of the stent wires being attached to the sleeve.

In a preferred embodiment, the sleeve can be adjusted or operated, such, that its length can be varied, thus varying the length of the stent wires attached thereto. This variation can be effected, e.g., via a rotation of the sleeve or via a click mechanism lengthening or shortening the length of the sleeve.

In yet another embodiment, the prosthetic valve device comprises at least a third, a fourth valve being attached within the lumen of stent frame and allowing unidirectional blood flow from a *vena cava* into the atrium. This way, the mechanical forces, i.e. the pressure, exerted to each of the valves can be reduced, such that the lifespan of the valves, and, thus, the one of the prosthetic valve device is enhanced and the tricuspid regurgitation is more efficiently treated.

According to one aspect of the invention, in the prosthetic valve device as claimed and disclosed, the valve is a biological valve comprising one, two, three or more leaflets.

The healthy human tricuspid valve comprises three leaflets, or cusps, named after their positions: anterior, posterior and septal. Thus, according to one aspect, the valve of the stented valve mounted on the stent graft member also comprises three leaflets, and thus, represents a tricuspid valve, whilst also a valve having only two leaflets and, having, thus, a "bicuspid" architecture, or with even one leaflet, i.e. a monocuspid valve, can be used with the prosthetic valve device according to the invention. Such valves can be created from human or animal donors. They can be created, e.g., from pericardium of human or any mammal, or from native leaflets from the heart or veins, or from any other biological material suitable for the intended purpose. Generally speaking, such valves are also called biological or tissue valves - as contrary to mechanical valves.

Accordingly, in a preferred embodiment, the biological valve comprises or consists of a material that is selected from animal pericardium, in particular porcine, bovine, equine pericardium, or from native leaflets from human heart or veins.

In another embodiment, the biological valve comprises or consists of a polymeric material.

In an aspect of the invention, the prosthetic valve device is covered at least partially by prosthetic material. The prosthetic material can be attached to the stent frame either from the outside of the stent frame or from the inside, i.e., from the luminal side, of the stent frame or from both sides.

Any suitable fluid-tight or substantially fluid-tight prosthetic material can be used. In a preferred embodiment, the prosthetic material is a biocompatible fabric, including but not limited to woven, knitted or otherwise fabricated material, such as polyester, such as polyethylene terephthalate, fluorinated polymers, such as polytetrafluoroethylene (PTFE), expanded PTFE (ePTFE), polyvinylidene fluoride, polysiloxane, including polydimethyl siloxane, polyurethanes, including polyetherurethanes, polylactide, polyglycolide and copolymers thereof.

Also, materials that are not inherently biocompatible may be used when previously subjected to surface modifications in order to render the materials biocompatible.

Further, biomaterial as mammal (i.e., bovine, porcine) derived pericardium or submucosa of small intestine (SIS) or any other cross-linked bio-material tissue suitable to be used as a graft material may be employed.

In another aspect of the invention, the stent frame comprises 4, 5, 6, 7, 8, 9 or 10 stent rings. This number of stent rings has shown to be sufficient to achieve the necessary radial expansion force to allow fixation while maintaining a compliance-allowing degree of flexibility.

In another aspect of the invention, the stent frame comprises at least two, preferably 2, 3, 4, 5, 6, 7 or 8 struts interconnecting the at least two stent rings in the uncovered section. Thus, the stent frame comprises at least two, preferably 2, 3, 4, 5, 6, 7 or 8 open cells, which are preferably of a hexagonal shape as discussed above.

The portions of the prosthetic valve device, i.e. the proximal, the medium and the distal portion and the first and any further valve can be variously sized (i.e.: length, diameter, etc.) as suitable for an intended use and as depending on the respective condition of the patient's heart, and are preferably larger in diameter than the inner vessel diameter the members of the prosthetic valve device are to be placed in.

According to an aspect of the prosthetic valve device of the invention, a diameter D1 of the distal and/or proximal portion, i.e. the *superior vena cava* portion and the *inferior vena cava* portion, preferably in an outermost proximal section or distalmost section of the proximal/distal portion, is between 20 to 50 mm, preferably between 22 to 48 mm, wherein the respective end numbers of these ranges are included.

According to a preferred embodiment of the prosthetic valve device comprising a sleeve, the medium portion, i.e. the uncovered or atrial section, has a length or height H of between 40 to 120 mm. According to a preferred embodiment, this length or height H can be varied through the sleeve as mentioned above.

According to another aspect of the prosthetic valve device of the invention, the proximal portion carrying the valve in their respective lumen, has a tulip shape, or, in other words, a shape where an outer proximal section OS of the proximal portion has a larger diameter D1 than a inner proximal section IS located adjacent to the outer proximal section OS towards the direction to the medium portion, which carries the valve, and which has a diameter D2. Also, the distal portion can have a tulip shape, or in other words, a shape where an outer distal section OS of the distal portion has a larger diameter D1 than an inner distal section IS located adjacent to the outer distal section OS towards the direction to the medium portion, which has a diameter D2. Preferably, the valves, if present in one or both of the proximal and distal portion, are located within the inner sections of the proximal and distal portions. This embodiment has the advantage that, via the smaller diameter D2 in these proximal and/or distal sections, a smaller valve can be provided and attached in the lumen at these sections.

According to another aspect of the invention, the stent frame is self-expanding, wherein the prosthetic valve device is configured, such, that it is convertible from a compressed state for introducing the prosthetic valve device into a heart of a mammal to an expanded state within the heart.

The mammal is preferably a human.

According to a preferred embodiment, the prosthetic valve device of the invention, along its longitudinal axis, varies in its diameter. With this embodiment, the different diameters D1, D2, and conditions of the vessels, in particular the *vena cava* can be respected.

Further, in a preferred embodiment, the diameter of the proximal portion is, in the expanded state of the prosthetic valve device, smaller than the diameter of the distal portion.

In this embodiment, i.e. where the proximal portion has - in its expanded state - a smaller diameter than the distal portion, the diameters are reflecting the larger diameter of the *inferior vena cava* as compared to the diameter of the *superior vena cava.* Thus, a secure fixation of the prosthetic valve device in the *vena cava* can be achieved.

In another embodiment of the prosthetic valve device according to the invention, the diameter of the prosthetic valve device varies along the respective length of the portions.

With this embodiment, an even finer adjustment of the prosthetic valve device to the respective vessel conditions and forms can be guaranteed.

The invention also relates to the use of the prosthetic valve device according to the invention for treating tricuspid regurgitation in a mammal, as well as to a method for treating tricuspid regurgitation in a mammal, comprising the step of delivering and/or implanting a device according to the invention to a position within the heart of a patient in need thereof in order to replace or support the native tricuspid valve of said patient.

The prosthetic valve device according to the invention can be either surgically implanted or delivered by transcatheter methods. In the latter case, i.e. with a transcatheter method, the prosthetic valve device according to the invention is loaded onto a suitable deployment catheter, there being compressed by a retractable sheath or tube. The deployment catheter is inserted into the heart of a patient whose tricuspid valve needs replacement or support. The deployment catheter having the prosthetic valve device according to the invention loaded thereon in a compressed state, is advanced via the jugular vein into the *superior vena cava* into the right atrium and into the *inferior vena cava,* as far as the distal end of the distal portion of the main body is placed into the *inferior vena cava,* the medium portion of the main body, within the right atrium and the proximal end of the proximal portion of the main body into the *superior vena cava.* Alternatively, the deployment catheter having the prosthetic valve device according to the invention loaded thereon in a compressed state can be advanced via the femoral vein into the *inferior vena cava* into the right atrium and into the *superior vena cava,* as far as the proximal end of the proximal portion of the main body is placed into the *superior vena cava,* the medium portion of the main body, within the right atrium and the distal end of the distal portion of the main body into the *inferior vena cava.* Correct placement can be monitored via the visualization elements.

Upon correct placement, the sheath or the otherwise compressing means is retracted to stepwise release the prosthetic valve device according to the invention, upon which action the stent members of the prosthetic valve device can expand and fixate the prosthetic valve device in the *superior vena cava* and *inferior vena cava,* respectively.

The one or more valve mounted on the prosthetic valve device can operate as soon as the compressing means are retracted.

Further advantages and features of the invention are set forth in the following description and in the attached figures.

It will be understood that the aforementioned features and the features still to be explained below can be used not only in the respectively specified combination but also in other combinations or on their own, without departing from the scope of the present invention.

The aforementioned features of the invention and the features still to be explained below are shown in the figures, in which:
- Fig. 1: shows a schematic drawing of a human heart;
- Fig. 2: shows a schematic drawing of an exemplary embodiment of the prosthetic valve device according to the invention placed in the correct position in the heart of Fig. 1;
- Fig. 3: shows a schematic drawing of an exemplary embodiment the prosthetic valve device according to the invention, outside the heart and in enlarged detail; and
- Fig. 4: shows an enlarged schematic drawing of the medium portion of an exemplary embodiment of the prosthetic valve device according to the invention.

Fig. 5 shows another embodiment of the prosthetic valve device according to the invention; A: in assembled form; B: with a part of the stent frame of the prosthetic valve device shown prior to final assembly of the prosthetic valve device; and C: an enlarged view of the medium portion of the prosthetic valve device showing the sleeve.

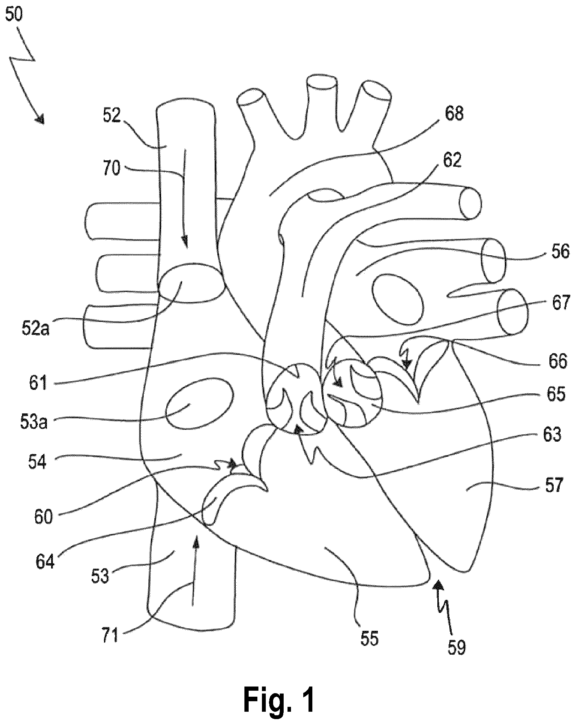

In Fig. 1, a human heart 50 is depicted, having a right atrium 54, a right ventricle 55, a left atrium 56 and a left ventricle 57. Also depicted in fig. 1 is a portion of the *superior vena cava* 52, entering the heart 50 via the right atrium 54, and a portion of the *inferior vena cava* 53. Arrows 70 and 71 indicate the physiological direction of blood flow.

In more detail, the *superior vena cava* 52 returns the blood from the upper half of the body and opens into the upper and back part of the right atrium 54, the direction of its orifice 52a being downward and forward. Its orifice 52a has no valve.

The *inferior vena cava* 53, which has a larger diameter than the *superior vena cava* 52, returns the blood from the lower half of the body, and opens into the lowest part of the right atrium 54, its orifice 53a being directed upward and backward, and guarded by a rudimentary valve, the valve of the *inferior vena cava* (Eustachian valve, not shown).

The right ventricle 55 has a triangular in form and extends from the right atrium 54 to near the apex 59 of the heart 50.

The right atrioventricular orifice (not depicted in fig. 1) is the large oval aperture of communication between the right atrium 54 and ventricle 55, and is guarded by the tricuspid valve 60.

The opening 61 of the pulmonary artery 62 is circular in form and is placed above and to the left of the atrioventricular opening; it is guarded by the pulmonary valves 63.

The tricuspid valve 60 consists of three about triangular cusps or segments or leaflets 64, the anterior, posterior and medial or septal cusp. Their bases are attached to a fibrous ring (not depicted in fig. 1) surrounding the atrioventricular orifice and are also joined to each other so as to form a continuous annular membrane. Their atrial surfaces are directed toward the blood current from the atrium 54, while their ventricular surfaces are directed toward the wall of the ventricle 55; together with the apices and margins of the cusps, they give attachment for the *chordae tendineae* (not depicted in fig. 1).

As discussed above, the function of the tricuspid valve is to prevent back flow of blood into the right atrium 54; arrows 70 and 71 indicate normal blood flow into the right atrium 54.

The left atrium 56 is smaller than the right atrium 54. The left ventricle 57 is longer and more conical in shape than the right ventricle 55. The left atrioventricular opening (mitral orifice, not depicted in fig. 1) is placed to the left of the aortic orifice 65, and is guarded by the bicuspid or mitral valve 66.

The aortic opening 65 is a circular aperture, in front and to the right of the atrioventricular opening, and its orifice is guarded by the three aortic valves 67. Reference number 68 designates the aorta.

Tricuspid regurgitation is not uncommon in the tricuspid valve 60, and means that blood from the right ventricle 55 flows back into the right atrium 54 upon contraction of the right ventricle 55 due to the tricuspid valve 60 not properly closing.

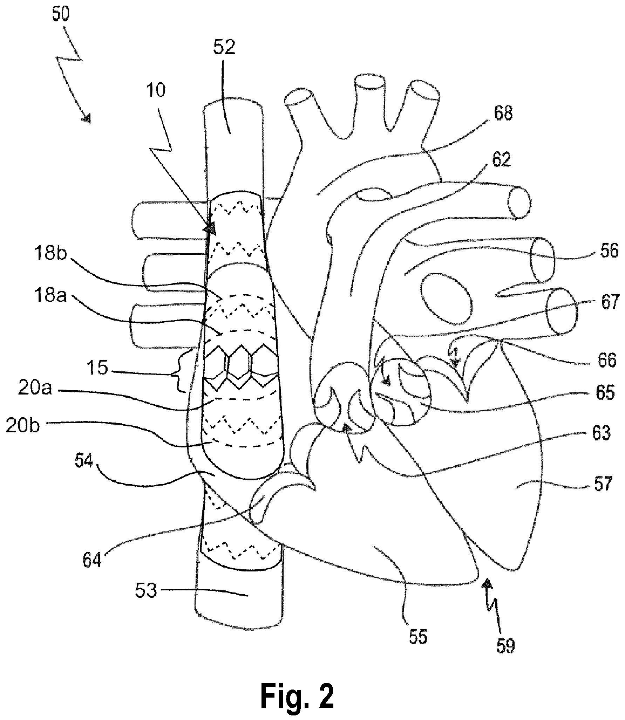

With the prosthetic valve device according to the invention, tricuspid regurgitation is to be treated, and placement of an exemplary embodiment of the prosthetic valve device according to the invention is depicted in the attached fig. 2.

Fig. 2 shows the schematic drawing of the heart as already depicted in fig. 1. For better understanding, Fig. 2 does not include all of the reference numbers as designated in fig. 1, but is meant to show the same features of the human heart 50.

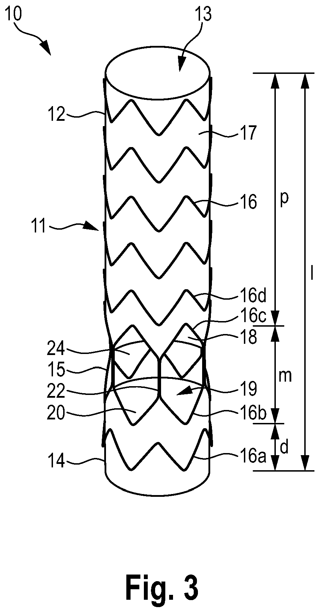

As can be seen in Fig. 2, the prosthetic valve device 10 according to the invention is placed in the expanded state in the human heart 50. The prosthetic valve device as such is shown in more detail in Fig. 3, and in the following it will be made reference to both, Fig. 2 and 3; for the sake of better understanding, not all of the features of the prosthetic valve device designated in Fig. 3 are designated in Fig. 2, however, the features are nevertheless the same.

Device 10 comprises a main body 11 with a length I, a lumen 13 permitting blood flow there through and a general tubular shape. The prosthetic valve device 10 is subdivided into a proximal portion 12 with a length p, a distal portion 14 with a length d and a medium portion 15 with a length m.

The specific length of each of the main body's portions can differ such that the uncovered medium portion may be provided centrally on the main body, i.e. length p and length d can be equal or substantially equal as shown in Fig. 2, or the length p and the length d may be different, as shown in Fig. 3 for length p > length d. Preferably, the medium portion is positioned closer to the distal end of the prosthetic valve device 10.

The prosthetic valve device 10 further comprises a tubular flexible stent frame 16 that is covered partially by prosthetic material 17 and that comprises at least a first valve 18.

The length l of the main body 11 is such that the proximal portion 12 can be, with regard to its length p partially inserted into the *superior vena cava* 52, while the distal portion 14 can be, with regard to its length d partially inserted into the *inferior vena cava* 53, such that the prosthetic valve device 10 is securely anchored within the *superior vena cava* 52 and *inferior vena cava* 53.

The diameter of the stent frame within the proximal portion 12 is such that it is slightly larger than the diameter of the *superior vena cava* 52. The diameter of the stent frame within the distal portion 14 is such that it is slightly larger than the diameter of the *inferior vena cava* 53. The prosthetic valve device 10 is to be positioned such that the medium portion is placed within the atrium.

In the embodiments shown in Fig. 2 to 4, which all comprise two or more valves, the prosthetic material covers the proximal and the distal portion. The medium portion 15 is uncovered and thus in permanent fluid-communication with the atrium 54. The prosthetic material 17 is made of or comprises a biocompatible material and provides together with the first and second valve 18 in the proximal portion 12 for a fluid-tight or substantially fluid-tight closure of the proximal portion 12 and distal portion 14 within the atrium 54. It is to be noted that in another embodiment, comprising a single first valve 18 in the proximal portion 12, but no second valve 20 (not shown), the distal portion 14 can be uncovered. However, in such embodiments it is to be noted that in all uncovered stent portions the rings need to be interconnected by stent structures as struts for example.

The arrow P in Fig. 3 designates the proximal direction, or, in other words, a direction towards the proximal portion 12, and the arrow D designates the distal direction, or, in other words, a direction towards the distal portion 14.

The first valve 18 can be provided directly adjacent to the uncovered medium portion 15 within the lumen 13 of the proximal portion 12, as indicated by reference sign 18a or can be provided more proximally as indicated by reference sign 18b. Alternatively, multiple valves can be provided at each of the positions 18a, 18b or elsewhere within the proximal portion.

The second valve 20 can be provided directly adjacent to the uncovered medium portion 15 within the lumen 13 of the distal portion 14, as indicated by reference sign 20a or can be provided more distally as indicated by reference sign 20b. Alternatively, multiple valves can be provided at each of the positions.

In the embodiment shown in Fig. 3, the valves 18 and 20 are a biological trileaflet valves. However, also embodiments with a bileaflet or monoleaflet valve or a valve with more than three leaflets are disclosed in the general section (not shown). The at least one valve may be created from human or animal pericardium or from native valves or veins or similar and represents a unidirectional valve, allowing blood to flow from the *vena cava* 52/53 into the right atrium 54 while blocking the blood flow in the opposite direction, i.e. blocks flow from the right ventricle 55 into the caval system.

In the embodiment shown in Fig. 3, the tubular flexible stent frame 16 comprises or consists of eight individual stent rings and the rings are not interconnected in the proximal portion 12 or distal portion 14 other than by the prosthetic material 17. In the medium portion 15, two rings 16b and 16c are interconnected by stent structures. It is preferred that the rings meander circumferentially and are disposed successively in the main body's longitudinal axis/direction, wherein the metal rings have a Z-shaped profile with pointed arches 25 and 26 pointing alternately toward the proximal end and distal end of the prosthetic valve device 10. This is especially preferred within the medium portion 15 since such an embodiment facilitates an interconnection.

In the embodiment illustrated in Fig. 2 and Fig. 3, the medium portion 15 comprises two interconnected rings. The two individual rings are interconnected via six struts 22 and are circumferentially meandering such, that they have a Z-shaped profile with six pointed arches 25 and 26 pointing alternately towards the proximal portion 12 and the distal portion 14 of the prosthetic valve device 10. The six struts 22 in the medium section 15 connect an arch of a first stent ring 16b pointing towards the proximal portion with an arch pointing towards the distal portion of a second stent ring 16c, which second stent ring 16c is arranged distally from the first stent ring 16b. Thus, the rings 16b and 16c together with the struts form openings 24 that are uncovered. Thus, the medium portion 15 is in fluid-communication with the atrium 54.

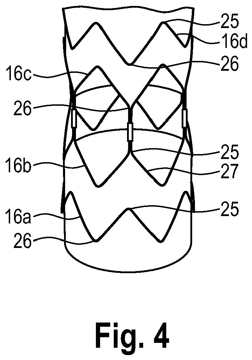

Fig. 4 illustrates an enlarged drawing of another embodiment, wherein the medium portion 15 comprises six regular hexagonal stent structures 27 connected via two opposed legs to a leg from another hexagonal stent structure. The resulting cyclic stent structure is attached to the proximal portion 12 and distal portion 14 via the prosthetic material covering of the proximal and distal portion. The openings 24 formed by the hexagonal stent structures are uncovered. Thus, the medium portion 15 is in fluid-communication with the atrium 54.

A can be seen in Fig. 4, the stent rings 16a and 16b (i.e., a first and a second stent ring) are arranged over the length l, such, that the pointed arches 25 of stent ring 16a, which point towards the proximal direction P, are aligned with those pointed arches 25 of stent ring 16b, which also point towards the proximal direction P, wherein the stent rings 16a, 16b are adjacent to one another and arranged at a certain distance DS from each other, so that the stent rings do not directly touch each other and are not interconnected with one another.

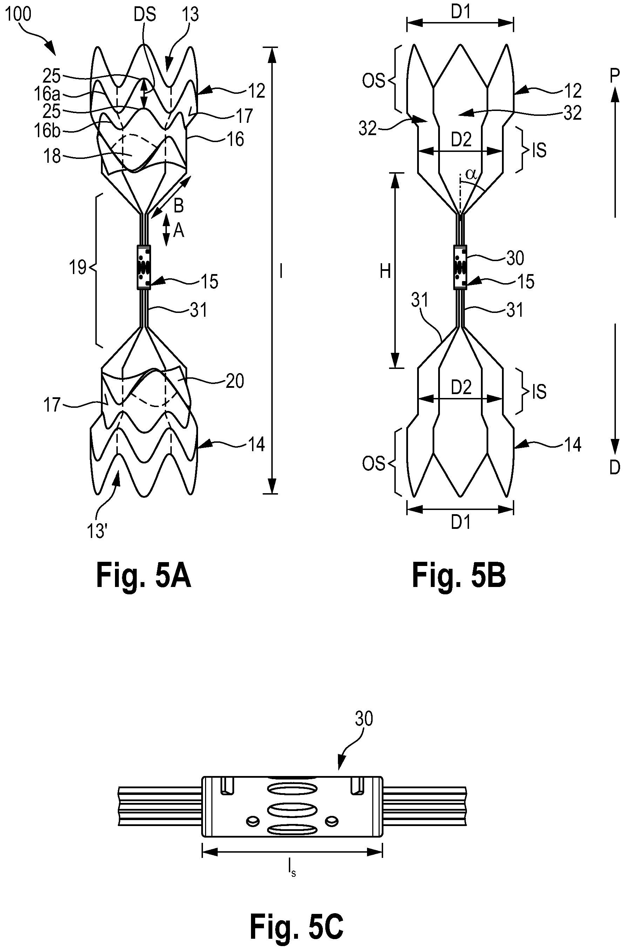

In Fig. 5, a second embodiment of the prosthetic valve device of the invention is shown, here designated with the reference number 100. Also this embodiment of the prosthetic valve device 100 comprises a length l and has a proximal portion 12 and a distal portion 14. Via the proximal portion 12, the of the prosthetic valve device 100 is anchored in the *superior vena cava,* and the distal portion 14 serves to anchor the of the prosthetic valve device 100 in the *inferior vena cava.* A medium portion 15 is interposed between the proximal portion 12 and the distal portion 14. As can be seen from Fig. 5A, which shows the embodiment in the assembled and expanded state, the prosthetic valve device 100 comprises a stent frame 16 extending from the proximal portion 12, via the medium portion 15, to the distal portion 14. In the proximal and distal portions 12, 14, the stent frame 16 is covered by a prosthetic material 17, while the medium portion 15 is free from the prosthetic material, and, thus, forms an uncovered section 19. The proximal and distal portions 12, 14 are, thus, tubular and comprise respective lumen 13, 13'.

Within the lumen 13 of the proximal portion 12 and within the lumen 13' of the distal portion 14, a first valve 18 and a second valve 20 are arranged/attached, respectively.

Upon implantation into the heart of a patient, the medium portion 15 is positioned within the right atrium of the heart, so that due to the uncovered section 19, the medium portion 15 allows blood flow out from the lumen 13; 13' into the right atrium.

In the proximal portion 12 and distal portion 14, the stent frame comprises circumferentially meandering stent rings 16 (two of which are exemplarily designated with 16a, 16b).

As can be seen in Fig. 5B, the stent frame of this embodiment not only comprises circumferentially meandering individual stent rings 16, but further a plurality of stent or nitinol wires 31: the stent wires 31 extend, in the medium portion 15, in a first section A substantially parallel to the longitudinal axis or length l of the prosthetic valve device 100, and in a second section B at an angle α, outwardly from the longitudinal axis. The stent wires are being held together or bundled or fixed via a sleeve 30, which is shown in more detail in Fig. 5C.

According to an alternative embodiment, the stent wires 31 directly extend outwardly from the sleeve 30 at an angle α, with reference to the longitudinal axis or length l of the prosthetic valve device 100 (not shown here).

The stent wires 31, respectively extend into the proximal and distal portion 12, 14, where they form bent loop-portions 32. These bent loop-portions 32 are arranged to form the tubular shape of the proximal and distal portions 12, 14. To this "basic" stent frame 16, circumferentially meandering stent rings 16a, 16b can be attached in the proximal and distal portion 12, 14, as well as the prosthetic material 17 and the two valves 18, 20.

Also, it has to be noted - generally and without explicit reference to figure 5, but which also shows this embodiment - that the stent wires 31 can extend either only towards the proximal direction P and only towards the distal direction D, such, or in other words, that a first plurality of stent wires 31 extends into the proximal direction P, and a second plurality of stent wires extends into the distal direction D.

As mentioned above, the proximal and distal portion 12, 14, as can be taken from Fig. 5A, each comprises circumferentially meandering stent rings 16. The circumferentially meandering stent rings 16 comprise pointed arches 25, 26, which alternately point into the distal direction D and proximal direction P.

As can be seen in Fig. 5A, also in the embodiment shown here, the stent rings 16a and 16b (i.e., a first and a second stent ring) are arranged over the length I in the proximal portion, such, that the pointed arches 25 of stent ring 16a, which point towards the proximal direction P, are aligned with those pointed arches 25 of stent ring 16b, which also point towards the proximal direction P, wherein the stent rings 16a, 16b are adjacent to one another and arranged at a certain distance DS from each other, so that the stent rings do not directly touch each other. In the embodiment shown in Fig. 5A, the stent rings 16 are attached to the loop-portions 32.

Fig. 5B shows the structure of the proximal and distal portion 12, 14 of the embodiment shown in Fig. 5 in more detail. From Fig. 5B it can be taken that a basic structure of the proximal and distal portion 12, 14 is formed in tulip-like shape, wherein each of the proximal portion 12 and distal portion 14 comprises a first or outer section OS, which represents the outermost section of the proximal portion 12 and distal portion 14, respectively, and a second or inner section IS, which represents the innermost section of the proximal and distal portion 12, 14, with respect to the medium portion 15.

As can be seen in Fig. 5B, the outer sections OS have a diameter D1 that is larger than the diameter D2 of the inner sections IS, thus forming the tulip-shape of the proximal and distal portions 12, 14. Preferably, the valves, if present in both of the proximal and distal portions 12, 14 are attached within the inner sections IS of the proximal and distal portions 12, 14.

Fig. 5C shows the sleeve 30 in an enlarged view. The sleeve 30 has the purpose to "bundle" the stent wires 31 and, where applicable, to provide attachment means for the stent wires 31 within the sleeve 30. Also, the sleeve 30 has a length Iₛ, which can be varied, e.g., via a rotation of parts of the sleeve, or via a click-mechanism.

## Claims

1. A prosthetic valve device (10; 100) for treatment of tricuspid valve insufficiency of a heart of a mammal, the prosthetic valve device (10; 100) comprising, along a length I:
a proximal portion (12) for anchoring the prosthetic valve device in the *superior vena cava,* a distal portion (14) for anchoring the prosthetic valve device in the *inferior vena cava,* and a medium portion (15) interposed between the proximal (12) and the distal (14) portion,
a stent frame (16) extending from the proximal portion (12) over the medium portion (15) to the distal portion (14), wherein the proximal portion (12) and the distal portion (14) are tubular and have a respective lumen (13; 13'), and wherein in the proximal portion (12) and the distal portion (14) the stent frame (16) is covered by prosthetic material (17) at least partially, and wherein the prosthetic valve device (10; 100) further comprises
a first valve (18) being attached within the lumen (13; 13') of the proximal portion (12), and, optionally, a second valve (20) being attached within the lumen (13') of the distal portion (14),
wherein the medium portion (15) is circumferentially free from prosthetic material (17), thus forming an uncovered section (19), and wherein the medium portion (15), in an implanted state of the prosthetic valve device (10; 100), is configured for being positioned within the right atrium of the heart, and for allowing blood flow out from the lumen (13; 13') into the right atrium.

2. The prosthetic valve device (10; 100) of claim 1 and 2, wherein the stent frame (16) comprises or consists of a laser-cut stent frame, a plurality of individual stent rings (16a, 16b, 16c, 16d), individual stent wires (31), or a woven or braided stent frame comprising interwoven or braided stent elements, or combinations thereof.

3. The prosthetic valve device (10; 100) of any of claim 1 or 2, wherein the stent frame (16; 16') has a plurality individual stent rings (16a, 16b, 16c, 16d), which are successively arranged over the length l of the prosthetic valve device (10; 100), and which circumferentially meander, the plurality of stent rings (16a, 16b, 16c, 16d) not being interconnected with one another via struts (22), or being at least partially interconnected with one another via struts (22).

4. The prosthetic valve device (10; 100) of any of any of claims 1 to 3, wherein the stent frame (16) has a plurality of individual stent rings (16a, 16b, 16c, 16d), which are successively arranged over a length l of the prosthetic valve device and which circumferentially meander, such, that the stent rings (16a, 16b, 16c, 16d), in the proximal (12) and distal portion (14) are not interconnected with one another via struts, and wherein the stent rings (16b, 16c), in the medium portion (15) are interconnected via struts.

5. The prosthetic valve device (10) of any of claims 1 to 4, wherein the uncovered section (19) of the medium portion (15) is formed by at least two stent rings (16b, 16c) that are positioned at a distance from each other in the medium portion (15) and that are interconnected via struts (22), such, that open cells (24) between the interconnected stent rings (16b, 16c) are formed in the medium portion (15), wherein the open cells (24) are dimensioned and sized to allow blood flow out from the lumen (13; 13') into the right atrium.

6. The prosthetic valve device (10) of claim 5, wherein the at least two stent rings (16b, 16c) in the medium portion (15) are circumferentially meandering, such, that they have a Z-shaped profile with pointed arches (25, 26) pointing alternately toward the proximal portion (12) and the distal portion (14) of the prosthetic valve device (10), and that the struts (22) in the medium portion (15) connect an arch (26) of a first stent ring (16c) pointing towards the distal portion (14) with an arch (25) pointing towards the proximal portion (12) of a second stent ring (16b), which second stent ring (16b) is arranged distally from the first stent ring (16c).

7. The prosthetic valve device (100) of any of claims 1 to 3, wherein the stent frame (16) is formed of
a plurality of single or individual straight, non-meandering stent wires (31) extending from the proximal portion (12) to the distal portion (14), wherein the stent wires (31), in the medium portion (15) are being held together via a sleeve (30), thus extending substantially parallel to the longitudinal length l of the prosthetic valve device (100), and wherein the single stent wires, in the proximal portion (12) and the distal portion (14) form bent loop-portions (32), respectively, which are arranged to form the tubular shape of the proximal (12) and distal portion (14).

8. The prosthetic valve device (100) of claim 7, wherein the stent frame further comprises a plurality of individual stent rings (16a, 16b) circumferentially meandering and being attached to the bent loop-portions in the proximal portion (12) and the distal portion (14).

9. The prosthetic valve device (100) of any of claims 1 to 3, wherein the stent frame (16) in the proximal portion (12) and the distal portion (14) is formed by circumferentially meandering stent rings (16), optionally interconnected with one another, and the stent frame (16) in the medium portion (15) is formed by straight, non-meandering stent wires (31) being connected with the stent frame (16) of the proximal portion (12) and the distal portion (14), the stent wires, in the medium portion (15) being held together via and attached within a sleeve (30).

10. The prosthetic valve device (10; 100) of any of the preceding claims, further comprising at least a third or a fourth valve being attached within the lumen of the proximal (12) or distal (14) portion of the prosthetic valve device (100).

11. The prosthetic valve device of any of the preceding claims, wherein the first (18) and/or the second valve (20) is a biological valve comprising one, two, three or more leaflets.

12. The prosthetic valve device of claim 11, wherein the biological valve comprises or consists of a material that is selected from animal pericardium, in particular porcine, bovine, equine pericardium, or from native leaflets from human heart or veins.

13. The prosthetic valve device (10) of any of the preceding claims, wherein the stent frame (16) comprises between 4 and 10 stent rings (16a, 16b, 16c, 16d).

14. The prosthetic valve device (10) of any claims 4 to 6, wherein the stent frame (16) comprises at least two, preferably between two and eight, struts interconnecting the stent rings (16b, 16c) in the uncovered section (19).

15. The prosthetic valve device (10) of any claim 5 or 6, wherein the uncovered section (19) comprises at least two, preferably between two and eight, open cells (24), which are preferably hexagonally shaped.
